# EUROPEAN PATENT SPECIFICATION

(11) **EP 2 549 000 B1**
(45) Date of publication and mention of the grant of the patent: **17.08.2016**
(21) Application number: 11756195.1
(22) Date of filing: 11.03.2011
(51) Int. Cl.: D01F 6/46, D04H 3/00, D01F 1/10

(54) **FIBER, NON-WOVEN FABRIC AND APPLICATION THEREOF**
FASER, VLIESSTOFF UND VERWENDUNG DAVON
FIBRE, NON-TISSÉ ET SON APPLICATION

(30) Priority: 15.03.2010 JP 2010058077
(43) Date of publication of application: 23.01.2013
(73) Proprietor: Mitsui Chemicals, Inc., Tokyo 105-7117 (JP)
(72) Inventor: ICHIKAWA, Taro, Sodegaura-shi Chiba 299-0265 (JP); KAWAKAMI, Yoshihisa, Sodegaura-shi Chiba 299-0265 (JP); TOMITA, Yoshihiko, Sodegaura-shi Chiba 299-0265 (JP)
(74) Representative: Hoffmann Eitle
(86) International application number: PCT/JP2011/055768
(87) International publication number: WO 2011/115009

(56) References cited:
- WO-A1-03/068856
- JP-A- 2000 080 522
- JP-A- 2001 295 131
- JP-A- 2009 040 896
- JP-A- 2010 013 761
- JP-A- 2011 063 911
- JP-A- 2011 089 224
- US-A- 4 070 218

## Description

### Technical Field

The present invention relates to a fiber which has good spinnability, with which smoke emission during spinning is suppressed, and which is excellent in initial hydrophilic property and enduring hydrophilic property, and to a nonwoven fabric containing such a fiber.

### Background Art

Nonwoven fabrics obtained from olefin polymers typified by polypropylene have widely been used in various applications because of being excellent in permeability, flexibility, and lightweight property. Therefore, for the nonwoven fabrics, various characteristics corresponding to their applications have been demanded and improvement in the characteristics has been needed.

On the other hand, the nonwoven fabrics comprising olefin polymers are necessarily subjected to hydrophilization treatment, for use in the topsheets of hygiene materials such as paper diapers and sanitary napkins, the wipers, and the like because of being essentially hydrophobic.

As one of methods of hydrophilization treatment of olefin polymers, a method of using a di-fatty acid ester of polyoxyethylene having an average molecular weight in a certain range (Patent Literature 1: JP 1997-503829 A or Patent Literature 2: JP 2006-508194 A) is proposed.

However, it was found that, when a dioleate ester of polyethylene oxide having an average molecular weight of 400 which is a diester based on an unsaturated carboxylic acid described in Example 1 and Example 5 of Patent Literature 1 is added to polypropylene to produce a spunbond nonwoven fabric, much smoke emission occurs during spinning, the initial hydrophilic property of the obtained spunbond nonwoven fabric is greatly inferior, and any hydrophilic property is not obtained at all unless "activation" is performed by heat treatment or the like.

On the other hand, the invention described in Patent Literature 2 is described to be used for soft-finishing of the fibers of polypropylene spunbond nonwoven fabrics and the like, not to be directed at hydrophilization treatment. In addition, it is described in the paragraph [0014] of the Japanese Unexamined Patent Application Publication that use of a reaction product of polyethylene glycol (molecular weight of 400) with decanoic acid (carbon number: 10) or lauric acid (carbon number: 12) is particularly preferable.

It was found that, in the spunbond nonwoven fabric obtained by adding a di-lauric acid ester of polyethylene glycol having an average molecular weight of 400 to polypropylene, as described in

Patent Literature 2, although initial hydrophilic and enduring hydrophilic properties as well as flexibility are good, much smoke emission occurs during spinning by extrusion forming at high temperature, a production facility is soiled with components from the smoke emission, and, therefore, it was difficult to maintain stable production.

### Citation List

### Patent Literature

Patent Literature 1: JP 1997-503829 A
Patent Literature 2: JP 2006-508194 A
Patent Literature 3: WO 03/068856 A1 relates to the use of compounds of general formulas (I) A-B-C-B-A and/or (II) A-B-A, in which: A represents a radical R-COO, whereby R represents a saturated, branched or unbranched alklyl radical having 7 to 21 C atoms; B represents a group (CₙH₂ₙO)ₖ, whereby n represents integers ranging from 2 to 4 and k can have a value ranging from 1 to 15, and; C represents a linear or branched alkylene radical having at least 2 but no more than 6 C atoms. These compounds are used as internal additives for carrying out the softening finishing of synthetic fibers that contain polyolefins.
Patent Literature 4: US 4070218 relates to a soft, nonwoven web that is produced by adding directly to a thermoplastic polymer at the time of extrusion a lubricating agent having an HLB number in the range of 8 to 20 and a molecular weight in the range from 200 to 4000. The lubricating agent is uniformly distributed into the polymer as extruded into filaments. The filaments are collected to form a web and then subjected to heat treatment in the range from 180-260 °F for at least about 1-7 seconds. The lubricating agent migrates to the surface of the fibers producing a release effect and preventing secondary bonding from occuring. After pattern bonding to provide spaced areas of high intensity bonds, the result is a soft, strong nonwoven web having particular utility as a liner for disposable diapers and catamenial devices.

### Summary of the Invention

### Problem to be Solved by the Invention

The present inventors performed examination in various ways for the purpose of: developing a fiber with which smoke emission during production of a nonwoven fabric is reduced and which is favorable in an olefin polymer nonwoven fabric that is excellent in smoke resistance and that is excellent in both initial hydrophilic property and enduring hydrophilic property, wherein the hydrophilic property (initial hydrophilic property) is expressed in short time after the production of the nonwoven fabric and the hydrophilic property (enduring hydrophilic property) is maintained with or without heat treatment; and developing a nonwoven fabric. As a result, the present inventors found that an object of the present invention can be achieved by adding and mixing, for a hydrophilization agent, a di-saturated fatty acid ester of a specific poly(oxyalkylene) diol with a poly(oxyalkylene) sorbitan tri-fatty acid ester as a nonionic surfactant.

### Means for Solving the Problem

The present invention is to provide a fiber comprising an olefin polymer composition comprising, based on 100 parts by weight of an olefin polymer, 0.5 to 5 parts by weight of a di-fatty acid ester of a poly(oxyalkylene) diol represented by formula (1) described below and 0.01 to 2.5 parts by weight of a poly(oxyalkylene) sorbitan tri-fatty acid ester (the alkylene group being ethylene, propylene or butylene group, the number of added oxyalkylene units being 10 to 40, and the fatty acid being an alkyl or alkylene fatty acid having a carbon number of 12 to 18).

R¹-COO-(CₙH₂ₙO)ₐ-C₆H₁₂-O(CₙH₂ₙO)_{b}-CO-R² (Formula 1)

[wherein R¹ and R² represent an alkyl group having a carbon number of 11 to 17 and may be the same or different, n in (CₙH₂ₙO)ₐ and (CₙH₂ₙO)_{b} is an integer of 2 to 4, and a + b is 5 to 23].

### Advantageous Effects of the Invention

The olefin polymer composition comprising a di-fatty acid ester of a specific poly(oxyalkylene) diol and a poly(oxyalkylene) sorbitan tri-fatty acid ester in accordance with the present invention has such characteristics that spinnability during spinning of the fiber of a nonwoven fabric or the like is excellent; smoke emission is suppressed; moreover, both initial hydrophilic property and enduring hydrophilic property are excellent, wherein the obtained fiber and nonwoven fabric have hydrophilic properties expressed for as extremely short as 3 hours or less after the production, and the hydrophilic properties are also maintained over heat treatment at 80°C for 2 hours.

### Description of the preferred Embodiments

### <Olefin Polymers>

Olefin polymers forming the fiber according to the present invention and a nonwoven fabric comprising the fiber are homo- or copolymers of alpha-olefins, such as ethylene, propylene, 1-butene, 1-hexene, 4-methyl-1-pentene, and 1-octene, and specifically include an ethylene polymer such as a homopolymer of ethylene or an ethylene/alpha-olefin copolymer, such as high-pressure low-density polyethylene, linear low density polyethylene (so-called LLDPE), high density polyethylene, an ethylene/propylene random copolymer, or an ethylene/1-butene random copolymer; a propylene polymer such as a homopolymer of propylene (so-called polypropylene), a propylene/ethylene random copolymer, a propylene/ethylene/1-butene random copolymer (so-called random polypropylene), a propylene block copolymer, or a propylene/1-butene random copolymer; a 1-butene polymer such as a 1-butene homopolymer, a 1-butene/ethylene copolymer, or a 1-butene/propylene copolymer; poly 4-methyl-1-pentene; or the like.

Of these olefin polymers, propylene polymers are preferred because a nonwoven fabric excellent in spinning stability during shape forming, the workability of the nonwoven fabric, permeability, flexibility, lightweight property, and thermal resistance can be obtained.

The olefin polymers according to the present invention may be blended, as needed, with an additive such as an auxiliary agent for promoting or suppressing hydrophilic properties, an antioxidant, a weathering stabilizer, a light stabilizer, an antiblocking agent, a lubricant, a nucleating agent, a pigment, a softening agent, a water repellency agent, a filler, or an antibacterial agent, each of which is typically used; or a polymer other than the olefin polymers, as long as the objects of the present invention are not compromised.

### <Propylene Polymers>

The propylene polymers according to the present invention are propylene homopolymers or copolymers of propylene with small amounts of one or two or more alpha-olefins having a carbon number of 2 or more (provided that a carbon number of 3 is excluded), preferably 2 to 8 (provided that a carbon number of 3 is excluded), such as ethylene, 1-butene, 1-pentene, 1-hexene, 1-octene, and 4-methyl-1-pentene, having a melting point (Tm) of 125°C or more, preferably in the range of 130 to 165°C.

The propylene polymers according to the present invention have a melt flow rate (MFR: ASTM D-1238, 230°C, load of 2160 g), without limitation, in the range of typically 1 to 1000 g/10 min, preferably 5 to 500 g/10 min, further preferably 10 to 100 g/10 min, as long as melt spinning can be performed.

Of the propylene polymers according to the present invention, propylene/alpha-olefin random copolymers of propylene with small amounts of one or two or more alpha-olefins having a carbon number of 2 or more, preferably 2 to 8, such as ethylene, 1-butene, 1-pentene, 1-hexene, 1-octene, and 4-methyl-1-pentene, having a melting point (Tm) ranging from 125 to 155°C, preferably from 130 to 147°C, are particularly preferred since a nonwoven fabric excellent in flexibility and initial hydrophilic property can be obtained.

The content of an alpha-olefin in the propylene/alpha-olefin random copolymer is typically in the range of 1 to 10 mol%, without limitation as long as the melting point (Tm) is in the above-described range. More preferably, it is 1 to 5 mol%.

<Di-saturated Fatty Acid Ester of Poly(oxyalkylene) Diol>

The di-saturated fatty acid ester of the poly(oxyalkylene) diol according to the present invention is represented by the formula (1) described below.

R¹-COO-(CₙH₂ₙO)ₐ-C₆H₁₂-O(CₙH₂ₙO)_{b}-CO-R² (Formula 1)

[wherein R¹ and R² represent an alkyl group having a carbon number of 11 to 17 and may be the same or different, n in (CₙH₂ₙO)ₐ and (CₙH₂ₙO)_{b} is an integer of 2 to 4, and a + b is 5 to 23].
In the formula (1), R¹ and R² groups are specifically CH₃-(CH₂)₁₁ group (dodecyl group), CH₃-(CH₂)₁₂ group (tridecyl group), CH₃-(CH₂)₁₃ group (tetradecyl group), CH₃-(CH₂)₁₄ group (pentadecyl group), CH₃-(CH₂)₁₅ group (hexadecyl group), CH₃-(CH₂)₁₆ group (heptadecyl group), and CH₃-(CH₂)₁₇ group (octadecyl group).

The di-saturated fatty acid ester of the poly (oxyalkylene) diol according to the present invention can be obtained by various known production methods, for example, by adding ethylene oxide, propylene oxide, butylene oxide, and the like to 1,6-hexanediol, followed by being diesterified with saturated fatty acids but is not limited thereto as long as the formula (1) is satisfied.

In consideration of initial hydrophilic properties, R¹ and R² groups are preferably CH₃-(CH₂)₁₁ group (dodecyl group), CH₃-(CH₂)₁₂ group (tridecyl group), CH₃-(CH₂)₁₃ group (tetradecyl group), CH₃-(CH₂)₁₄ group (pentadecyl group), and CH₃-(CH₂)₁₅ group (hexadecyl group).

In the case of the di-saturated fatty acid ester of the poly(oxyalkylene) diol having CH₃-(CH₂)₈ group (nonyl group) as R having a carbon number of 9, smoke emission during spinning is severe and an obtained nonwoven fabric is inferior in hydrophilic property, particularly enduring hydrophilic property. On the other hand, in the case of the di-saturated fatty acid ester of the poly (oxyalkylene) diol having CH₃-(CH₂)₁₇ group (octadecyl group) as R having a carbon number of 18 or CH₃-(CH₂)₇CH=CH(CH₂)₇ group, an obtained nonwoven fabric is inferior in initial hydrophilic property and enduring hydrophilic property although smoke emission during spinning is suppressed.

In the formula (1), the (CₙH₂ₙO)ₐ and (CₙH₂ₙO)_{b} groups are specifically adducts of ethylene oxide, propylene oxide, and butylene oxide, preferably ethylene oxide adducts, the number of added oxyalkylene units being 5 to 23, preferably 10 to 15.

Di-fatty acid esters of which the number of added oxyalkylene units of the (CₙH₂ₙO)ₐ and (CₙH₂ₙO)_{b} groups, i.e., a + b is less than 5 are not preferred for stable production since much smoke emission occurs during shape forming. Further, there is a fear of elution of a hydrophilization agent component over time when an obtained nonwoven fabric is immersed in water. Much elution of di-fatty acid esters into water is not preferred since in contact with the water during production and use, its residual water may become clouded to require special treatment for drainage. Further, its use as the topsheet of a diaper or the like is not preferred since there is a fear of flow of di-fatty acid esters into its surroundings during the use to soil the skin or to hydrophilize another member to be originally hydrophobic. On the other hand, in the case of di-fatty acid esters with a + b of more than 23, there is a fear in which the hydrophilic property of an obtained nonwoven fabric is insufficient or poor filling of an olefin polymer composition becomes significant to prevent good fibers and nonwoven fabrics from being obtained when the olefin polymer composition is introduced into an extruder to be molten and formed although smoke emission during shape forming and elution of a hydrophilization agent into water hardly occur.

On the other hand, a mono-saturated fatty acid ester of a poly(oxyalkylene) diol represented by R-COO-(CₙH₂ₙO)-H is inferior in extruding formability, so that good fibers and nonwoven fabrics cannot be obtained. It is not preferred for stable production since much smoke emission during shape forming occurs even when the shape forming can be performed.

### <Poly(oxyalkylene) Sorbitan/Tri-fatty Acid Ester>

The alkylene group of the poly(oxyalkylene) sorbitan/tri-fatty acid ester according to the present invention is ethylene, propylene or butylene group, the number of added oxyalkylene units is 10 to 40, and the fatty acid is an alkyl or alkylene fatty acid having a carbon number of 12 to 18.

The sorbitan ring of the poly(oxyalkylene) sorbitan/tri-fatty acid ester according to the present invention is not limited to a 5-membered ring or a 6-membered ring, and a site of addition of an oxyalkylene group or esterification is not also limited.

### <Olefin Polymer Composition>

The olefin polymer composition according to the present invention is a composition comprising, based on 100 parts by weight of the olefin polymer, 0.5 to 5 parts by weight, preferably 1 to 3 parts by weight, of the di-saturated fatty acid ester of the poly(oxyalkylene) diol, and 0.01 to 2.5 parts by weight, preferably 0.5 to 2.0 parts by weight, of the poly(oxyalkylene) sorbitan/tri-fatty acid ester.

When the amount of the di-saturated fatty acid ester of the poly (oxyalkylene) diol is less than 0.5 part by weight, the hydrophilic property is inferior. In contrast, when it is more than 5 parts by weight although the upper limit is not particularly limited, the hydrophilic property is saturated, and the amount of the di-saturated fatty acid ester of the poly (oxyalkylene) diol which exudes to the surfaces of an obtained fiber and a nonwoven fabric containing the fiber is increased to deteriorate forming workability.

The amount of the poly(oxyalkylene) sorbitan/tri-fatty acid ester of less than 0.01 part by weight is not preferred since a synergistic effect with the di-saturated fatty acid ester of the poly(oxyalkylene) diol is not obtained and poor filling of a raw material becomes significant to prevent good fibers and nonwoven fabrics from being obtained when an olefin polymer composition is introduced into an extruder to be molten and formed.

In contrast, the amount of the poly(oxyalkylene) sorbitan/tri-fatty acid ester of more than 2.5 parts by weight is not preferred, although the upper limit is not particularly limited, since the hydrophilic property is inferior.

When the olefin polymer composition according to the present invention is prepared, a master batch in pellet form comprising more than 5% by weight, for example, 10 to 70% by weight of the di-saturated fatty acid ester of the poly (oxyalkylene) diol at high concentration is preferably used since the addition of the di-saturated fatty acid ester of the poly(oxyalkylene) diol and the poly (oxyalkylene) sorbitan/tri-fatty acid ester, and the mixing with the olefin polymers during production of the fiber and the nonwoven fabric are facilitated. An olefin polymer which is used in the master batch of the mixture of the di-saturated fatty acid ester of the poly(oxyalkylene) diol with the poly(oxyalkylene) sorbitan/tri-fatty acid ester is not limited to the olefin polymers described above but other olefin polymers may also be used. As the olefin polymer used in such a master batch, MFR may appropriately be selected depending on a fiber of interest and a nonwoven fabric containing the fiber.

The olefin polymer composition according to the present invention may be blended, as needed, with an additive such as an antioxidant, a weathering stabilizer, a light stabilizer, an antiblocking agent, a lubricant, a nucleating agent, or a pigment, each of which is typically used; or a polymer other than the olefin polymers, as long as the obj ects of the present invention are not compromised.

### <Fiber and Nonwoven Fabric>

The fiber according to the present invention and the nonwoven fabric containing the fiber are a fiber of the olefin polymer composition comprising the olefin polymers, the di-saturated fatty acid ester of the poly(oxyalkylene) diol, and the poly(oxyalkylene) sorbitan/tri-fatty acid ester; and a nonwoven fabric containing the fiber.

The fiber according to the present invention and the nonwoven fabric containing the fiber are excellent in initial hydrophilic property, and when an initial hydrophilic property becomes poor by a combination of the olefin polymer used with the di-saturated fatty acid ester of the poly(oxyalkylene) diol and the poly(oxyalkylene) sorbitan/tri-fatty acid ester, the hydrophilic property can be expressed by heat treatment of the obtained nonwoven fabric. As the conditions of the heat treatment in this case, it is generally preferably performed for around 5 minutes to 2 hours at a temperature of 40 to 90°C, depending on the properties of the olefin polymer used.

The fiber according to the present invention typically has a fineness ranging from 0.5 to 5 denier. The fiber according to the present invention is preferably a filament since falling or the like of the fiber from the obtained nonwoven fabric does not occur, although it may also be a staple fiber.

The cross-sectional shape of the fiber according to the present invention may be a heteromorphic cross section such as a V-shape, a cruciate shape, or a T-shape, as well as a round shape.

The fiber according to the present invention may be a side-by-side-type crimped fiber comprising the composition including at least two olefin polymers. Preferably, mention may be made of side-by-side-type crimped fibers comprising propylene homopolymers and propylene/ethylene random copolymers as olefin polymers.

The fiber according to the present invention may also be a concentric or eccentric fiber and, in this case, the di-saturated fatty acid ester of the poly(oxyalkylene) diol and the poly(oxyalkylene) sorbitan/tri-fatty acid ester may also be added to both core and sheath or either thereof.

Furthermore, the fiber according to the present invention, in combination with another fiber, may form a filament-mixed nonwoven fabric. For example, the fiber according to the present invention may form a filament-mixed nonwoven fabric of such a crimped fiber with another non-crimped fiber.

The nonwoven fabric according to the present invention may also be entangled by various known entangling methods, for example, by a method using means such as a needle punch, a water jet, or ultrasonic waves, or by a method for partial thermal fusion bonding by hot embossing using an emboss roll or by using a hot air through, depending on its application. Such an entangling method may be used singly or in combination of a plurality of entangling methods.

When the thermal fusion bonding is performed by hot embossing, an embossment area proportion is typically in the range of 5 to 30%, preferably 5 to 20%. A mark shape is exemplified by circle, oval, ellipse, square, rhombus, rectangle, quadrangle, kilt, lattice, and tortoise-shell shapes and continuous shapes based on those shapes.

The nonwoven fabric according to the present invention may be used singly or by lamination with another layer depending on various applications.

Other layers laminated with the nonwoven fabric according to the present invention may specifically include, for example, knitted fabrics, woven fabrics, nonwoven fabrics, films, paper-products. When the nonwoven fabric according to the present invention and another layer are laminated (affixed), various known methods including thermal fusion bonding methods such as hot embossing and ultrasonic wave fusion bonding, mechanical entangling methods such as needle punches and water jets, methods with adhesives such as hot-melt adhesives and urethane-based adhesives, extruded laminates, and the like may be adopted. The nonwoven fabric according to the present invention is highly convenient for controlling the quality of products since hydrophilic properties are maintained even after the step of contacting with a high-temperature substance such as a hot-melt adhesive or an extruded laminate.

Nonwoven fabrics laminated with the nonwoven fabric according to the present invention may include various known nonwoven fabrics such as other spunbond nonwoven fabrics, melt blow nonwoven fabrics, wet-laid nonwoven fabrics, dry-laid nonwoven fabrics, air-laid pulp nonwoven fabrics, flash-spun nonwoven fabrics, and split yarn nonwoven fabrics.

Materials constituting such nonwoven fabrics may be exemplified by various known thermoplastic resins, for example, polyolefins which are homo- or copolymers of alpha-olefins such as ethylene, propylene, 1-butene, 1-hexene, 4-methyl-1-pentene, and 1-octene, such as high-pressure low-density polyethylene, linear low density polyethylene (so-called LLDPE), high density polyethylene, polypropylene, polypropylene random copolymers, poly 1-butene, poly 4-methyl-1-pentene, ethylene/propylene random copolymers, ethylene/1-butene random copolymers, and propylene/1-butene random copolymers; polyesters (such as polyethylene terephthalate, polybutylene terephthalate, and polyethylene naphthalate), polyamides (such as nylon-6, nylon-66, and polymetaxylene adipamide), polyvinyl chloride, polyimide, ethylene/vinyl acetate copolymers, polyacrylonitrile, polycarbonate, polystyrene, ionomers, and thermoplastic polyurethane, and mixtures thereof. Of these, high-pressure low-density polyethylene, linear low density polyethylene (so-called LLDPE), high density polyethylene, polypropylene, polypropylene random copolymers, polyethylene terephthalate, polyamides, and the like are preferred.

When a nonwoven fabric comprising a crimped fiber is used as another spunbond nonwoven fabric laminated with the nonwoven fabric according to the present invention, an obtained laminated product is excellent in flexibility, bulk property, and feeling. Further, when a stretching-worked nonwoven fabric comprising a blend fiber of the respective filaments of a thermoplastic resin and a thermoplastic elastomer is used, an obtained laminated product is excellent in contraction and expansion property in addition to the above-described characteristics.

When the nonwoven fabric according to the present invention is used in an absorbent article, for example, the topsheet or second sheet of a disposable diaper, or a sheet (core wrap) which wraps an absorbent material, a fineness ranging from 0.5 to 3 denier and a basis weight ranging from 7 to 50 g/m² are preferred.

When the nonwoven fabric according to the present invention is used as the sheet (core wrap) which wraps the absorbent material of the absorbent article, it may also be used by lamination with the melt blown nonwoven fabric.

As a film laminated with the nonwoven fabric according to the present invention, a permeable (moisture vapor permeable) film which makes use of hydrophilic properties which are the characteristics of the nonwoven fabric according to the present invention is preferred. Such permeable films may include various known permeable films, for example, films comprising thermoplastic elastomers such as polyurethane elastomers, polyester elastomers, and polyamide elastomers having moisture vapor permeability, porous films prepared by stretching films comprising thermoplastic resins containing inorganic or organic fine particles to be porous, and the like. Thermoplastic resins used in the porous films are preferably polyolefins such as high-pressure low-density polyethylene, linear low density polyethylene (so-called LLDPE), high density polyethylene, polypropylene, polypropylene random copolymers, or compositions thereof.

The nonwoven fabric according to the present invention may also be used by being subjected to secondary working such as gear working, printing, coating, lamination, heat treatment, or size enlargement working as long as the objects of the invention are not compromised.

### <Method for Producing Nonwoven Fabric>

When a staple fiber is used as a raw material for a nonwoven fabric, its production can be performed by various known production methods, for example, a wet method or a dry method (card).

### <Method for Producing Filament Nonwoven Fabric>

The filament nonwoven fabric according to the present invention can be produced by various known methods for producing nonwoven fabrics, among which a method of production by a spunbond process is preferred in terms of excellent productivity.

When the filament nonwoven fabric according to the present invention is produced by the spunbond process, it can be produced by performing melting with one extruder at 180 to 250°C, preferably 190 to 230°C, in the case of production of a spunbond nonwoven fabric comprising a single fiber of the olefin polymer composition, discharging a fiber from a spinneret set at 180 to 250°C, preferably 190 to 230°C, having a spinning nozzle, at a single bore discharge amount of 0.4 to 1.5 g/min, preferably 0.5 to 0.8 g/min, spinning the fiber out, cooling the spun-out fiber with air for cooling at 10 to 40°C, preferably 20 to 40°C, pulling and thinning the fiber with high-speed air at 2000 to 7000 m/min, preferably 3000 to 6000 m/min to have a predetermined fineness, and trapping the fiber on a trapping belt to be deposited to have a predetermined thickness (basis weight), followed by entanglement of the fiber by an entangling method such as a method using means such as a needle punch, a water jet, or ultrasonic waves, or a method for partial thermal fusion bonding by hot embossing using an emboss roll or by using a hot air through.

### <Absorbent Article>

The absorbent article according to the present invention is an absorbent article prepared by using the nonwoven fabric, preferably the filament nonwoven fabric, as the topsheet and/or the second sheet, or the sheet (core wrap) which wraps an absorbent material. Examples of the absorbent article according to the present invention include disposable diapers, underpants or sanitary products, bladder control pads, and sheets for pets.

Particularly, in the case of use as the topsheet or second sheet of the disposable diaper, or the sheet (core wrap) which wraps an absorbent material, when the nonwoven fabric comprising the fiber of the olefin polymer composition is used, it is preferable to contain, based on 100 parts by weight of the olefin polymer, 0.5 to 5 parts by weight of a di-fatty acid ester of a poly(oxyalkylene) diol and 0.01 to 2.5 parts by weight of a poly(oxyalkylene) sorbitan/tri-fatty acid ester.

### Examples

The present invention is further specifically described below with reference to examples but is not limited to these examples.

Physical property values in Examples and Comparative Examples were measured by methods described below. Further, in measurement of (1) liquid flow (hydrophilic property), an aqueous solution of sodium chloride (9 g/liter) having a surface tension of 70 +/- 2 mN/m was used as artificial urine.

Further, the measurement of (1) liquid flow was performed on two conditions of: after a lapse of 24 hours but within 48 hours (without heat treatment) of production of a nonwoven fabric; and within 2 hours (with heat treatment) of taking out a nonwoven fabric heat-treated at a set temperature of 80°C for 2 hours after a lapse of 24 hours or more of production of the nonwoven fabric.

### (1) Liquid Flow

A sample (150 mm x 400 mm) was collected from a nonwoven fabric. A commercially available paper diaper from which a topsheet was removed was fixed on a plate tilted at 45° and fixed, and the sample was put on the same position as the location on which the removed topsheet had been present. From a height of about 10 mm of a sample surface, 80 ml of artificial urine was dropped with a rush through a 100 ml burette (fully opening the stopper of the burette), and a distance between the top of the drops flowing on the sample surface and the first drop point was measured. The shorter distance indicated a better hydrophilic property.

### (2) Extrusion Performance

An olefin polymer composition was charged into an extruder set at 200 °C to evaluate its extrusion state based on the following criteria.

o: A molten material is extruded while rotating a screw.

x: A molten material is not extruded but skidded in the extruder even by rotating a screw.

### (3) Smoke Emission Property

A smoke emission state from a nozzle portion when an olefin polymer composition molten at 200 °C by a spunbond process was spun was evaluated based on the following criteria.

o: A smoke emission state that is approximately equivalent to a state where a hydrophilization agent is not kneaded.

x: A state where significant smoke emission is observed or smoke emission is not recognized but it is required to stop spinning within 8 hours to clean a smoke emission component adhering to a spinning portion.

### (4) Basis Weight (g/m²)

Ten samples of 100 mm x 100 mm were collected from arbitrary points of a nonwoven fabric and the mass (g) of each was measured. The mean value thereof was determined and converted into mass per 1 m² as a basis weight (g/m²).

### [Example 1]

To 19 parts by weight of poly(oxyethylene)-1,6-hexanediol/dilaurate (diester-1) listed in Table 1, 1 part by weight of poly(oxyethylene) sorbitan/triolate (triester-1) listed in Table 1, and 80 parts by weight of a propylene/ethylene random copolymer (PP-1) having a melting point (Tm) of 142°C and an MFR of 60 g/10 min, 0.05 part by weight of an antioxidant (manufactured by Ciba Corporation, trade name: Irgafos 168) was added, and the resultant was melt-kneaded at 230°C and extruded to prepare a master batch in pellet form (hydrophilization agent-1).

Then, 7.5 parts by weight of the hydrophilization agent-1 was added to 92.5 parts by weight of propylene/ethylene random copolymer (PP-1) having a melting point (Tm) of 142°C and an MFR of 60 g/10 min, and the resultant was mixed to yield a propylene polymer composition (composition-1).

When the composition-1 was charged into a single screw extruder (screw diameter of 30 mm, L/D = 30) and heated and molten at an extrusion output of 4.4 kg/hr and a resin temperature of 200°C to evaluate extrusion performance, the filling state of the composition-1 into the extruder screw was good (o), similar to that in the case of adding no hydrophilization agent.

Then, the composition-1 was used and molten and spun by a spunbond process to obtain a filament nonwoven fabric. In this case, white smoke originating from the hydrophilization agent-1 was hardly observed from a spinning nozzle outlet, and this case was similar to the case without the addition. Following the spinning, hot embossing was performed to obtain a filament nonwoven fabric having a basis weight of 20 g/m². Then, using an emboss roll having a mark shape of a rhombus, an embossment area proportion of 18%, and an embossment area (area per mark) of 0.41 mm², hot embossing was performed at a temperatures of the emboss roll and a smoothing roll of 125°C and a linear pressure of 60 N/mm. The hydrophilic property was measured after a lapse of 24 hours but within 48 hours of production of the filament nonwoven fabric (without heat treatment). Further, a filament nonwoven fabric after a lapse of 24 hours or more of its production was hung in the vicinity of the center in an oven (manufactured by Espec Corp. , Tabai Safety Oven STS222) and heat-treated for 2 hours at a set temperature of 80°C, followed by performing similar measurement within 2 hours of taking out it (with heat treatment) .

The physical properties of the obtained filament nonwoven fabric were measured by the above-described method. The results are listed in Table 1.

### [Examples 2 to 5]

Filament nonwoven fabrics were obtained in the same manner as in Example 1 except that the amounts of the di-fatty acid ester-1 and the tri-fatty acid ester-1 used in Example 1 were changed as listed in Table 1. The physical properties and results of the obtained filament nonwoven fabrics are listed in Table 1.

### [Example 6]

A filament nonwoven fabric was obtained in the same manner as in Example 1 except that an alkyl group having a carbon number of 17 in R¹ and R² in the formula (1), listed in Table 1 (diester-2) was used, instead of the di-fatty acid ester-1 used in Example 1. The physical properties and results of the obtained filament nonwoven fabric are listed in Table 1.

**[Table 1]**

| | | Example 1 | Example 2 | Example 3 | Example 4 | Example 5 | Example 6 |
|---|---|---|---|---|---|---|---|
| Propylene polymer | | PP-1 | PP-1 | PP-1 | PP-1 | PP-1 | PP-1 |
| Diester | | Diester 1 | Diester 1 | Diester 1 | Diester 1 | Diester 1 | Diester 2 |
| | Diol | 1,6-hexanediol | 1,6-hexanediol | 1,6-hexanediol | 1,6-hexanediol | 1,6-hexanediol | 1,6-hexanediol |
| | a + b | 10 | 10 | 10 | 10 | 10 | 10 |
| | Carbon number of R¹ and R² | 11 | 11 | 11 | 11 | 11 | 17 |
| | Addition amount | 1.43 parts by weight | 2.85 parts by weight | 4.75 parts by weight | 2.93 parts by weight | 1.50 parts by weight | 2.85 parts by weight |
| Triester | | Triester 1 | Triester 1 | Triester 1 | Triester 1 | Triester 1 | Triester 1 |
| | The number of added oxyalkylene units of oxyethylene | 30 | 30 | 30 | 30 | 30 | 30 |
| | Carbon number of fatty acid | 17 | 17 | 17 | 17 | 17 | 17 |
| | Addition amount | 0.08 parts by weight | 0.15 parts by weight | 0.25 parts by weight | 0.08 parts by weight | 1.50 parts by weight | 0.15 parts by weight |
| Extrusion performance | | ○ | ○ | ○ | ○ | ○ | ○ |
| Smoke emission state | | ○ | ○ | ○ | ○ | ○ | ○ |
| Basis weight (g/m²) | | 20 | 20 | 20 | 20 | 20 | 20 |
| Hydrophilic property (nm: liquid flow method) without heat treatment | | 300 | 230 | 180 | 320 | 305 | No hydrophilic property |
| Hydrophilic property (mm: liquid flow method) with heat treatment | | 250 | 210 | 150 | 260 | 250 | 300 |

### [Comparative Example 1]

It was intended that a master batch in pellet form (hydrophilization agent-1) was prepared in the same manner as in

Example 1 except that only the diester-1 used in Example 1 was added and the triester-2 was not added, but kneading was not able to be performed to make subsequent shape forming impossible since the di-fatty acid ester-1 had a low affinity for the propylene polymer.

### [Comparative Example 2]

It was intended that a master batch in pellet form (hydrophilization agent-1) was prepared in the same manner as in Example 1 except that a diester-3 (the 1,6-hexanediol group in the formula (1) was changed to a 1,4-butanediol group) listed in Table 1 was added instead of the diester-1 used in Example 1 and the triester-1 was not added, but kneading was not able to be performed to make subsequent shape forming impossible since the hydrophilization agent had a low affinity for a resin.

### [Comparative Example 3]

Although a filament nonwoven fabric was obtained in the same manner as in Example 1 except that a diester-4 (alkyl group having a carbon number of 9 in R¹ and R² in the formula (1)) listed in Table 1 was used instead of the diester-1 used in Example 1, significant smoke emission during shape forming occurred and this case was x. The physical properties and results of the obtained filament nonwoven fabric are listed in Table 2.

### [Comparative Example 4]

Although a filament nonwoven fabric was obtained in the same manner as in Example 1 except that a diester-5 (in the formula (1), n is 2 and a + b = 3) listed in Table 1 was used instead of the di-fatty acid ester-1 used in Example 1, significant smoke emission during shape forming occurred and this case was x. The physical properties and results of the obtained filament nonwoven fabric are listed in Table 2.

### [Comparative Example 5]

Although production of a filament nonwoven fabric was attempted in the same manner as in Example 1 except that a di-fatty acid ester-6 (in the formula (1), n is 2 and a + b = 30) listed in Table 1 was used instead of the diester-1 used in Example 1, a molten material was not extruded and a propylene polymer composition was skidded in the extruder even when the screw of the extruder was rotated, so that the filament nonwoven fabric was not able to be obtained.

**[Table 2]**

| | | Comparative example 1 | Comparative example 2 | Comparative example 3 | Comparative example 4 | Comparative example 5 |
|---|---|---|---|---|---|---|
| Propylene polymer | | PP-1 | PP-1 | PP-1 | PP-1 | PP-1 |
| Diester | | Diester 1 | Diester 3 | Diester 4 | Diester 5 | Diester 6 |
| | Diol | 1,6-hexanediol | 1,4-butanediol | 1,6-hexanediol | 1,6-hexanediol | 1,4-butanediol |
| | a + b | 10 | 10 | 10 | 3 | 30 |
| | Carbon number of R¹ and R² | 11 | 11 | 9 | 11 | 11 |
| | Addition amount | 3.00 parts by weight | 3.00 parts by weight | 2.85 parts by weight | 2.85 parts by weight | 2.85 parts by weight |
| Triester | | - | - | Triester 1 | Triester 1 | Triester 1 |
| | The number of added oxyalkylene units of oxyethylene | - | - | 30 | 30 | 30 |
| | Carbon number of fatty acid | - | - | 17 | 17 | 17 |
| | Addition amount | - - | - | 0.15 parts by weight | 0.15 parts by weight | 0.15 parts by weight |
| Extrusion performance | | X | X | O | O | X |
| Smoke emission state | | - | - | X | X | - |
| Basis weight (g/m²) | | - | - | 20 | 20 | - |
| Hydrophilic property (mm: liquid flow method) without heat treatment | | - | - | 170 | No hydrophilic property | - |
| Hydrophilic property (mm: liquid flow method) with heat treatment | | - | - | 160 | No hydrophilic property | - |

### Industrial Applicability

In the fiber according to the present invention and the nonwoven fabric containing the fiber, smoke emission is suppressed during spinning the fiber, and, moreover, the obtained fiber and nonwoven fabric exhibit excellent initial hydrophilic properties and enduring hydrophilic properties and can preferably be used for a wide variety of applications as hygiene materials.

## Claims

1. A fiber comprising an olefin polymer composition comprising, based on 100 parts by weight of an olefin polymer, 0.5 to 5 parts by weight of a di-fatty acid ester of a poly (oxyalkylene) diol represented by formula (1) described below and 0.01 to 2.5 parts by weight of a poly(oxyalkylene) sorbitan tri-fatty acid ester wherein the alkylene group of the poly(oxyalkylene) sorbitan tri-fatty acid ester is an ethylene, propylene or butylene group, the number of added oxyalkylene units is from 10 to 40, and wherein the fatty acid is an alkyl or alkylene fatty acid having a carbon number of 12 to 18:
R¹-COO-(CₙH₂ₙO)ₐ-C₆H₁₂-O(CₙH₂ₙO)_{b}-CO-R² ··· (Formula 1)
wherein R¹ and R² represent an alkyl group having a carbon number of 11 to 17 and may be the same or different, n in (CₙH₂ₙO)ₐ and (CₙH₂ₙO)_{b} is an integer of 2 to 4, and a + b is 5 to 23.

2. The fiber according to claim 1, wherein the olefin polymer is a propylene polymer.

3. The fiber according to claim 2, wherein the propylene polymer is a propylene/alpha-olefin random copolymer having a melting point (Tm) ranging from 125 to 155°C.

4. The fiber according to claim 1, wherein, in the formula (1), n in (CₙH₂ₙO)ₐ and (CₙH₂ₙO)_{b} is 2.

5. The fiber according to claim 1, wherein, in the formula (1), R¹ and R² have a carbon number of 11 to 16.

6. The fiber according to any of claims 1 to 5, wherein the fiber is a filament.

7. A nonwoven fabric comprising the fiber according to any of claims 1 to 6.

8. An absorbent article prepared by using the nonwoven fabric according to claim 7 as a topsheet and/or a second sheet.

9. An absorbent article prepared by using the nonwoven fabric according to claim 7 as a sheet which wraps an absorbent material.

## Patentansprüche

1. Faser, umfassend eine Olefinpolymerzusammensetzung, umfassend, bezogen auf 100 Gew.-Teile eines Olefinpolymers, 0,5 bis 5 Gew.-Teile eines Di-Fettsäureesters eines durch die nachfolgende Formel (1) dargestellten Poly(oxyalkylen)diols und 0,01 bis 2,5 Gew.-Teile eines Poly(oxyalkylen)sorbitan-tri-Fettsäureesters, wobei die Alkylengruppe des Poly(oxyalkylen)sorbitan-tri-Fettsäureesters eine Ethylen-, Propylen- oder Butylengruppe ist, die Anzahl der hinzugefügten Oxyalkyleneinheiten 10 bis 40 ist, und wobei die Fettsäure eine Alkyl- oder Alkylen-Fettsäure mit einer Kohlenstoffanzahl von 12 bis 18 ist:
R¹-COO-(CₙH₂ₙO)ₐ-C₆H₁₂-O(CₙH₂ₙO)_{b}-CO-R² (Formel 1)
wobei R¹ und R² eine Alkylgruppe mit einer Kohlenstoffanzahl von 11 bis 17 darstellen und gleich oder verschieden sein können, n in (CₙH₂ₙO)ₐ und (CₙH₂ₙO)_{b} eine ganze Zahl von 2 bis 4 ist und a + b 5 bis 23 ist.

2. Faser gemäß Anspruch 1, wobei das Olefinpolymer ein Propylenpolymer ist.

3. Faser gemäß Anspruch 2, wobei das Propylenpolymer ein Propylen/alpha-Olefin-Random-Copolymer mit einem Schmelzpunkt (Tm) im Bereich von 125 bis 155°C ist.

4. Faser gemäß Anspruch 1, wobei, in der Formel (1), n in (CₙH₂ₙO)ₐ und (CₙH₂ₙO)_{b} 2 ist.

5. Faser gemäß Anspruch 1, wobei, in der Formel (1), R¹ und R² eine Kohlenstoffanzahl von 11 bis 16 aufweisen.

6. Faser gemäß einem der Ansprüche 1 bis 5, wobei die Faser ein Filament ist.

7. Vliesstoff, umfassend die Faser gemäß einem der Ansprüche 1 bis 6.

8. Absorptionsgegenstand, hergestellt unter Verwendung des Vliesstoffes gemäß Anspruch 7 als ein Oberblatt und/oder ein zweites Blatt.

9. Absorptionsgegenstand, hergestellt unter Verwendung des Vliesstoffes gemäß Anspruch 7 als eine Schicht, die ein Absorptionsmaterial umhüllt.

## Revendications

1. Fibre comprenant une composition de polymère oléfinique comprenant, sur la base de 100 parties en poids d'un polymère oléfinique, 0,5 à 5 parties en poids d'un diester d'acide gras d'un poly(oxyalkylène)diol représenté par la formule (1) décrite ci-dessous et 0,01 à 2,5 parties en poids d'un triester d'acide gras de poly(oxyalkylène)sorbitane, dans laquelle le groupe alkylène du triester d'acide gras de poly(oxyalkylène) sorbitane est un groupe éthylène, propylène ou butylène, le nombre de motifs d'oxyalkylène ajoutés est de 10 à 40, et dans laquelle l'acide gras est un acide gras d'alkyle ou d'alkylène ayant un nombre d'atomes de carbone de 12 à 18 :
R¹-COO-(CₙH₂ₙO)ₐ-C₆H₁₂-O(CₙH₂ₙO)_{b}-CO-R² ... (Formule 1)
dans laquelle R¹ et R² représentent un groupe alkyle ayant un nombre d'atomes de carbone de 11 à 17 et peuvent être identiques ou différents, n dans (CₙH₂ₙO)ₐ et (CₙH₂ₙO)_{b} est un nombre entier de 2 à 4, et a + b est 5 à 23.

2. Fibre selon la revendication 1, dans laquelle le polymère oléfinique est un polymère de propylène.

3. Fibre selon la revendication 2, dans laquelle le polymère de propylène est un copolymère statistique propylène/alpha-oléfinique ayant un point de fusion (Tm) allant de 125 à 155 °C.

4. Fibre selon la revendication 1, dans laquelle, dans la formule (1), n dans (CₙH₂ₙO)ₐ et (CₙH₂ₙO)_{b} est 2.

5. Fibre selon la revendication 1, dans laquelle, dans la formule (1), R¹ et R² ont un nombre d'atomes de carbone de 11 à 16.

6. Fibre selon l'une quelconque des revendications 1 à 5, dans laquelle la fibre est un filament.

7. Tissu non tissé comprenant la fibre selon l'une quelconque des revendications 1 à 6.

8. Article absorbant préparé en utilisant le tissu non tissé selon la revendication 7 comme feuille supérieure et/ou seconde feuille.

9. Article absorbant préparé en utilisant le tissu non tissé selon la revendication 7 comme feuille qui enveloppe un matériau absorbant.
